# EUROPÄISCHE PATENTSCHRIFT

(11) **EP 4 312 896 B1**
(45) Veröffentlichungstag und Bekanntmachung des Hinweises auf die Patenterteilung: **31.12.2025**
(21) Anmeldenummer: 22718606.1
(22) Anmeldetag: 24.03.2022
(51) Int. Cl.: A61F 2/60, A61F 2/68, A61F 2/76

(54) **PROTHESENSCHAFTSYSTEM UND VERFAHREN ZU DESSEN HERSTELLUNG**
PROSTHESIS SOCKET SYSTEM AND METHOD FOR PRODUCING SAME
SYSTÈME D'EMBOÎTURE DE PROTHÈSE ET SON PROCÉDÉ DE FABRICATION

(30) Priorität: 29.03.2021 DE 102021107815
(43) Veröffentlichungstag der Anmeldung: 07.02.2024
(73) Patentinhaber: Ottobock SE & Co. KGaA, 37115 Duderstadt (DE)
(72) Erfinder: KOPPE, Mario, 37115 Duderstadt (DE); FINKE, Lars Benjamin, 37115 Duderstadt (DE)
(74) Vertreter: Gramm, Lins & Partner Patent- und Rechtsanwälte PartGmbB
(86) Internationale Anmeldenummer: PCT/EP2022/057812
(87) Internationale Veröffentlichungsnummer: WO 2022/207461

(56) Entgegenhaltungen:
- US-A1- 2014 243 996
- US-A1- 2019 183 663

## Beschreibung

Die Erfindung betrifft ein Prothesenschaftsystem mit einem Grundkörper mit einer Schaftwand, die im angelegten Zustand eine Gliedmaße oder einen Gliedmaßenstumpf zumindest teilweise umgibt und zumindest zwei sich einander gegenüberliegende Schaftwandränder oder Schaftwandbereiche aufweist, sowie ein Verfahren zum Herstellen eines solchen Prothesenschaftsystems.

Ein Prothesenschaft dient zur Aufnahme einer Gliedmaße oder eines Gliedmaßenstumpfes, um weitere prothetische Komponenten, wie Gelenke, Aktuatoren, Sensoren, Steuerungseinrichtungen und künstliche Gliedmaße, wie Prothesenhände oder Prothesenfüße, an einem Patienten sicher festzulegen. Ein wesentlicher Punkt dabei ist, dass ein Prothesenschaft sicher an der Gliedmaße oder dem Gliedmaßenstumpf festliegt, ohne dass die Gefahr besteht, dass der Prothesenschaft sich ungewollt löst. Weiterhin stellt der Prothesenschaft die Orientierung der weiteren Prothesenkomponenten relativ zu der Gliedmaße oder dem Gliedmaßenstumpf sicher. Dazu ist es vorteilhaft, wenn der Prothesenschaft möglichst eng an der Gliedmaße oder dem Gliedmaßenstumpf anliegt. Zur Erhöhung des Tragekomforts und zur Beibehaltung einer definierten Schnittstelle wird beispielsweise die sogenannte Linertechnologie verwendet, bei der ein Prothesenliner zwischen den Gliedmaßenstumpf oder die Gliedmaße und dem Prothesenschaft angeordnet wird. Die Verriegelung zwischen dem Liner und dem Schaft kann über mechanische Komponenten oder über Unterdruck erfolgen. Für die sogenannte Vakuumschafttechnologie ist es notwendig, einen geschlossenen Prothesenschaft zu haben, der zumindest bereichsweise evakuierbar ist.

Prothesenschäfte werden entweder individuell anhand eines Modells oder direkt auf dem Stumpf angeformt oder bestehen aus Streben oder Wandkomponenten, die verlagerbar zueinander an einem distalen Endstück befestigt sind. Über Gurte oder andere Einrichtungen ist es möglich, die Streben oder Wandkomponenten relativ zueinander zu verlagern.

Aus der DE 10 2007 025 410 A1 ist ein Prothesenschaft zur Aufnahme eines Amputationsstumpfes und einer Extremität mit Anschlussmitteln für eine distale Protheseneinrichtung bekannt, wobei der Prothesenschaft zumindest eine Schale aufweist, die einen gekrümmten, offenen Querschnitt aufweist und deren Schalenenden im angelegten Zustand einander zumindest teilweise überlappen. Zumindest ein Spannmittel ist an der Schale angeordnet, das in Umfangsrichtung wirksam ist und die Schalenenden zueinander verspannt.

Aus der EP 1 411 872 B1 ist eine Prothese mit einem Prothesenliner mit Kupplungsstift und einem Prothesenliner mit Längsschlitzen bekannt, an dem ein Halter zum Verbinden einer künstlichen Gliedmaße mit dem Prothesenschaft angeordnet ist. Der Prothesenschaft besitzt einen konzentrischen Bund, in dem ein zylindrischer Adapter höhenverstellbar befestigt ist. Die Längsschlitze sind überbrückt und der Prothesenschaft kann mittels Spannelementen im Durchmesser verändert werden.

Aus der EP 1 555 967 B1 sind ein Prothesenschaft mit einem Sensor zum Messen eines Druckes zwischen dem Prothesenschaft und dem Amputationsstumpf sowie ein Sensor zum Messen von Kraft, Temperatur und/oder Feuchtigkeit bekannt. Die Sensoren sind mit einem Display oder einer Warneinrichtung verbunden. Werden Grenzwerte für Sensorwerte überschritten, wird ein Warnsignal ausgegeben.

Aus der EP 3 454 792 B1 ist ein Prothesenschaft mit einer proximalen Einführöffnung und einem einen Stumpf zumindest teilweise umgebenden Innenumfang, mit zumindest einer Anschlussvorrichtung für eine Prothesenkomponente, die an dem Prothesenschaft befestigbar ist, mit zumindest einem Aktuator, über den der Innenumfang des Prothesenschaftes veränderbar ist, und mit einer Steuerung, die mit zumindest einem Sensor gekoppelt ist, bekannt, wobei der Sensor als Inertialsensor ausgebildet ist. Die Steuerungseinrichtung ist mit einem Aktuator verbunden, wobei der Aktuator in Abhängigkeit von den empfangenen Sensorsignalen aktiviert oder deaktiviert wird. An dem Prothesenschaft kann ein innenliegender Drucksensor und/oder Motorstromsensor zur Erfassung des durch ein Stützelement auf den Stumpf aufgebrachten Druckes an einem Stützelement angeordnet sein, wobei mehrere Stützelemente vorhanden sein können, die elastisch ausgebildet oder elastisch gelagert sind. Die Stützelemente können in Umfangsrichtung überlappend ausgebildet sein.

Die US2014/243996 A1 betrifft eine Protheseneinrichtung mit einem Außenschaft aus einem in Längserstreckung starren Material mit zumindest einem, zumindest teilweise in Längserstreckung verlaufende Schlitz, durch den es möglich ist, eine Verformung des Außenschaftes in Radialrichtung zu ermöglichen. Ein Innenschaft aus einem flexiblen Material ist in dem Außenschaft angeordnet. Zumindest eine Spanneinrichtung ist an dem Außenschaft festgelegt, über die eine Durchmesserveränderung des Außenschaftes erreichbar ist. Auf einer dem Innenschaft zugewandten Seite des Außenschaftes ist zumindest eine Elektrode verlagerbar befestigt.

Aufgabe der vorliegenden Erfindung ist es, ein Prothesenschaftsystem und ein Verfahren zu dessen Herstellung bereitzustellen, mit denen eine verbesserte Gesamtversorgung für den Patienten mit mechatronischen Komponenten bereitgestellt werden kann.

Erfindungsgemäß wird diese Aufgabe durch ein Prothesenschaftsystem mit den Merkmalen des Hauptanspruches und ein Verfahren mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das Prothesenschaftsystem mit einem Grundkörper mit einer Schaftwand, die im angelegten Zustand eine Gliedmaße oder einen Gliedmaßenstumpf zumindest teilweise umgibt und zumindest zwei sich einander gegenüberliegende Schaftwandränder oder Schaftwandbereiche aufweist, sieht zwei Anker vor, wobei je ein Anker in einem Schaftwandrand oder an einem Schaftwandbereich angeordnet ist sowie ein mechatronisches Funktionselement, das an den Ankern befestigt ist, insbesondere zwischen den Schaftwandrändern oder in einem Ausschnitt innerhalb der Schaftwand angeordnet ist, wobei das Funktionselement als Modul ausgebildet ist, das einen Bereich zwischen den einander gegenüberliegenden Schaftwandrändern überdeckt oder diesen ausfüllt. Über die Anker in einem Schaftwandrand oder an einem Schaftwandbereich wird eine definierte mechanische Schnittstelle zwischen dem mechatronischen Funktionselement und der Schaftwand oder den Schaftwandbereichen des Prothesenschaftsystems bereitgestellt. Die individuelle Ausgestaltung der zumindest einen Schaftwand wird über die Anker um ein mechatronisches Funktionselement ergänzt, das als vorgefertigtes Modul ausgebildet ist. Das mechatronische Funktionselement kann mit allen Komponenten aufeinander abgestimmt vorgefertigt und an der Schaftwand befestigt werden, um den Prothesenschaft zu komplettieren. Dadurch ist es nicht mehr notwendig, insbesondere Sensoren und weitere Komponenten zur Bereitstellung eines adaptiven Prothesenschaftes oder eines mechatronischen Gesamtkonzeptes der prothetischen Versorgung individuell in oder an einer Schaftwand zu befestigen und die einzelnen Komponenten individuell zu konfigurieren. In dem mechatronischen Funktionselement können alle elektrischen, elektronischen und mechanischen Schnittstellen und Komponenten vorab industriell gefertigt werden und integriert sein, wobei die Individualisierbarkeit des Prothesenschaftsystems nicht oder nur geringfügig eingeschränkt wird. Die Gestaltung der übrigen Schaftwand und der anderen Komponenten der Prothese liegt weiterhin bei dem Orthopädietechniker, dem mit dem System ein Bauelement mit maximaler, erprobter Zuverlässigkeit bei minimaler Baugröße bereitgestellt wird.

Eine Weiterbildung sieht vor, dass das mechatronische Funktionselement zumindest einen Energiespeicher, einen Sensor, eine Steuerungseinheit, eine Kommunikationsstelle und/oder einen Aktuator aufweist. Durch die Integration der mechanischen, elektrischen oder elektronischen Komponenten sowie der Datenverarbeitungseinrichtungen können alle notwendigen Baugruppen und eine zentrale Recheneinheit vorgefertigt und aufeinander abgestimmt in einem Modul integriert werden. Der zumindest eine Energiespeicher stellt eine integrierte Energieversorgung inklusive Laderegelung für das mechatronische Funktionselement bereit. Die elektrischen und elektronischen Komponenten können wasserdicht innerhalb des mechatronischen Funktionselementes angeordnet und untergebracht sein und mit einem ausreichenden mechanischen Schutz durch ein Gehäuse oder eine entsprechende Integration versehen sein. Der Aktuator ermöglicht es beispielsweise, die Anker aufeinander zu oder voneinander weg zu bewegen, sodass die Schaftwand oder der Grundkörper verformt wird. Der Grundkörper oder die Schaftwand kann elastisch ausgebildet sein, sodass diese nach einer Verformung oder Verlagerung aus einem Ausgangszustand in diesen zurückkehrt. Die Schaftwand oder der Grundkörper kann zumindest bereichsweise flexibel sein und durch die Verlagerung der Anker durch den Aktuator oder die Aktuatoren in unterschiedliche Formen gebracht werden. Dadurch ist es beispielsweise möglich, eine verbesserte Anpassung der Schaftwand im angelegten Zustand an die Gliedmaße oder einen Gliedmaßenstumpf herzustellen.

Bevorzugt ist das mechatronische Funktionselement reversibel lösbar an den Ankern festlegbar ausgebildet, sodass wiederholt das Funktionselement zwischen den Schaftwandrändern oder den Schaftwandbereichen angeordnet und von dort wieder entfernt werden kann, ohne dass eine Zerstörung von Komponenten durchgeführt werden muss. Die reversible Festlegung kann beispielsweise über Formschlusselemente und/oder Kraftschlusselemente erfolgen, beispielsweise über Haken, Zapfen, Schienen, Klipse, Magnete, Klemmeinrichtungen und/oder andere mechanische oder magnetische Einrichtungen, die ein Befestigen und ein Lösen des mechatronischen Funktionselementes erlauben.

Das mechatronische Funktionselement ist zum Bewegen der Anker zueinander ausgebildet, insbesondere, um die Anker aufeinander zu und voneinander weg zu bewegen, um eine Volumenveränderung des Prothesenschaftes zu erreichen. Auch eine andere Relativverlagerung der Anker zueinander kann erfolgen, sodass eine Verlagerung der Schaftwand oder der Schaftwände zueinander durch das Aktivieren oder Deaktivieren des mechatronischen Funktionselementes erfolgen kann. Dadurch ist es möglich, eine Formanpassung und insbesondere eine Volumeneinstellung des Prothesenschaftes zu ermöglichen. Beispielsweise kann das Volumen vergrößert werden, um das Anziehen des Prothesenschafes zu erleichtern. Bei der Detektion von entsprechenden Belastungen oder Beschleunigungen kann das Volumen verringert werden, um einen sicheren Sitz des Prothesenschaftes zu gewährleisten.

Ebenso kann in Situationen, in denen eine Komforterhöhung gewünscht wird, beispielsweise beim Sitzen. Das mechatronische Funktionselement kann auch relativ zu den Ankern bewegbar ausgebildet sein, um eine Verlagerung des mechatronischen Funktionselementes an der Schaftwand oder relativ zu der Schaftwand zu ermöglichen. Auch ohne Änderung der Position der Anker zueinander kann durch Aktivierung des mechatronischen Funktionselementes die Position der Schaftwand verändert werden, beispielsweise um Sensoren an einem anderen Ort zu positionieren, eine Verlagerung einer Druckeinwirkung zu bewirken oder eine veränderte Steifigkeit durch die neue Positionierung des mechatronischen Funktionselementes vorzunehmen.

Die Anker sind vorteilhafterweise als Formschlusselemente und/oder Kraftschlusselemente ausgebildet, um eine reversible Festlegung des mechatronischen Funktionselementes an den Schaftwandrändern oder an der Schaftwand zu ermöglichen. Die Anker können beispielsweise als Schienen, Schienensegmente, Zapfen oder Löcher, gegebenenfalls auch als Bohrungen in der Schaftwand oder in dem Schaftwandbereich, ausgebildet sein. Über die Schienen kann eine formschlüssige Verriegelung durch Einschieben einer korrespondierenden Schiene oder Führung an dem mechatronischen Funktionselement erreicht werden. Die Schienen können als Schienensegmente ausgebildet sein und nur bereichsweise entlang der Schaftwandränder angeordnet oder ausgebildet sein. Ebenso können Formschlusselemente oder Vorsprünge oder Hinterschnitte in die Schienen eingeklipst werden, sodass keine schiebende Einführbewegung in Längserstreckung der Schiene, sondern eine Relativbewegung senkrecht dazu erfolgt. Ebenfalls kann eine klemmende, also kraftschlüssige Kopplung in Schienen oder Klemmelementen erfolgen. Über Magnete können zusätzliche Haltekräfte bereitgestellt werden und beispielsweise formschlüssige Sicherungselemente aus einer Entriegelungsstellung in eine Verriegelungsstellung gebracht werden, wenn eine gewünschte Endposition erreicht ist.

Die Anker können in oder an dem Grundkörper formschlüssig, kraftschlüssig und/oder stoffschlüssig befestigt sein. Alternativ können die Anker als Teil der Schaftwand, des Schaftwandrandes oder des Schaftwandrandbereiches ausgebildet sein. Die Anker können in der Schaftwand insbesondere eingegossen, eingeklemmt, eingehakt, eingeklebt, einlaminiert oder eingeschoben sein, ebenso kann der oder können die Anker an dem Grundkörper angeschraubt, angeschweißt, angenietet, angeklebt, anlaminiert, angenäht oder auf den Grundkörper aufgeschoben sein. Die Anker können auch als Bohrungen oder angeformte Vorsprünge oder Schlitze sowie Hinterschnitte ausgebildet sein.

Eine Variante sieht vor, dass der Grundkörper individuell geformt ausgebildet ist, beispielsweise direkt an der Gliedmaße oder an dem Gliedmaßenstumpf anmodelliert wird. Alternativ kann der Grundkörper aus vorgefertigten Modulen, entweder Standardmodulen oder individuell abgeformten Modulen ausgebildet sein oder als ein Standardgrundkörper mit verschiedenen Konfektionsgrößen ausgebildet sein. Die jeweils verwendeten Materialien werden entsprechend dem Fertigungsverfahren und den gewünschten Eigenschaften des Prothesenschaftes ausgewählt.

Die Schaftwandränder können durch ein Trennverfahren erzeugt sein, beispielsweise ausgeschnitten, ausgelasert, ausgestanzt oder auf andere Arten und Weisen trennend erzeugt werden. Sind beispielsweise schon freie Bereiche in dem Grundkörper vorhanden, können die Schaftwandränder durch Schleifen oder Fräsen so angepasst werden, dass das mechatronische Funktionselement zwischen den Schaftwandrändern positioniert oder zwischen den Ankern so festgelegt werden kann, so dass eine Festlegung des mechatronischen Funktionselementes an den einander gegenüberliegenden Schaftwandrändern erfolgt. Das mechatronische Funktionselement kann in einem Freiraum oder einem Ausschnitt zwischen den Schaftwandrändern so angeordnet sein, dass ein geschlossener oder im Wesentlichen geschlossener Querschnitt und damit ein vollständig umfänglich geschlossener Prothesenschaft erzielt werden kann.

Die Schaftwandränder sind beabstandet zueinander und zumindest abschnittsweise entlang aneinander ausgerichtet, also im Wesentlichen parallel zueinander oder mit leichten Fluchtungsfehlern zueinander. Alternativ können sich die Schaftwandränder konisch oder gekrümmt in Längserstreckung des Grundkörpers aufweiten, sodass ein Funktionselement, insbesondere durch Einführen in Proximal-Distal-Richtung, leicht zu den jeweiligen Ankern positioniert und an dem Grundkörper festgelegt werden kann.

Die Schaftwandränder verlaufen in einer Ausgestaltung in Proximal-Distal-Richtung des Prothesenschaftes oder sind schräg zu der Proximal-Distal-Richtung orientiert, wodurch die Montage von dem proximalen Schaftrand aus erleichtert wird.

Der Grundkörper kann als Spritzgussbauteil, aus thermoplastischem Material geformt, additiv gefertigt oder im Laminataufbau hergestellt sein. Bevorzugt ist eine im Wesentlichen konische Grundform des Grundkörpers, insbesondere mit einem offenen Querschnitt vorgesehen, wobei der offene Querschnitt oder die Ausnehmung zum Einführen des mechatronischen Funktionselementes entweder bei der Herstellung des Grundkörpers oder nachträglich erzeugt werden kann. Innerhalb einer konischen Grundform, sei es mit offenem Querschnitt oder mit einem geschlossenen Querschnitt, kann der Grundkörper zumindest einen Ausschnitt aufweisen, um darin das mechatronische Funktionselement aufzunehmen oder damit das mechatronische Funktionselement den Ausschnitt zumindest teilweise ausfüllen und/oder abdecken kann.

An dem Grundkörper kann eine Endkappe angeformt oder befestigt sein. Die Endkappe kann geschlossen oder teilgeschlossen ausgebildet sein und an dem distalen Ende Befestigungseinrichtungen für weitere Prothesenkomponenten aufweisen, insbesondere für Gelenkeinrichtungen, Überbrückungskomponenten wie Unterschenkelrohre oder Unterarmrohre, oder Prothesenunterschenkel oder Prothesenunterarme.

An der Endkappe sind in einer Ausführungsform Befestigungseinrichtungen für das mechatronische Funktionselement angeordnet, über die es möglich ist, das mechatronische Funktionselement mit der Endkappe zu verriegeln. Die Befestigungseinrichtungen können formschlüssig und/oder kraftschlüssig wirken und unterstützen insbesondere die korrekte Positionierung des mechatronischen Funktionselementes in oder an dem Grundkörper.

Die Schaftwandränder können elastisch zueinander verlagerbar ausgebildet sein, insbesondere können sie aufeinander zu und voneinander weg verlagert werden, um eine Volumenanpassung über einen Aktuator in dem mechatronischen Funktionselement zu ermöglichen. Aufgrund der elastischen Verlagerbarkeit wird die Rückstellung in eine Ausgangsposition durch die elastischen Rückstellkräfte erleichtert. Alternativ oder ergänzend ist die Schaftwand reversibel verformbar ausgebildet, sodass durch eine entsprechende Verlagerung der Anker aufgrund der Aktuatoren Bewegung unterschiedliche Formen oder Volumina einstellbar sind.

Eine Weiterbildung sieht vor, dass an dem mechatronischen Funktionselement Befestigungseinrichtungen für die Anker angeordnet oder ausgebildet sind, sodass das mechatronische Funktionselement an oder in den Ankern fixierbar ist. Die Befestigungseinrichtungen sind insbesondere korrespondierend ausgebildete Formschlusseinrichtungen, in die die Anker eingreifen oder mit denen die Anker in Eingriff treten, wenn das mechatronische Funktionselement montiert ist.

Das mechatronische Funktionselement ist in einer Ausgestaltung zwischen den Schaftwand Rändern, in einem Ausschnitt der Schaftwand oder an der Außenseite der Schaftwand angeordnet und ergänzt den Grundkörper zur Ausgestaltung eines vollständigen Prothesenschaftes.

Das Verfahren zum Herstellen eines Prothesenschaftsystems, wie es oben beschrieben wurde, sieht zunächst das Erzeugen eines Grundkörpers mit einer Schaftwand und das Erzeugen von Schaftwandrändern vor. Die Erzeugung der Schaftwandränder kann bei der Erzeugung des Grundkörpers erfolgen oder nachträglich vorgenommen werden. Weiterhin sind Anker an den Schaftwandrändern oder Schaftwandrandbereichen auszubilden oder zu befestigen. Die einstückige Ausgestaltung von Ankern erfolgt während der Erzeugung des Grundkörpers, werden separate Anker eingesetzt, werden diese an dem Grundkörper, insbesondere an den Schaftwandrändern oder Schaftwandrandbereichen befestigt. Die Anker können einander gegenüberliegen und beispielsweise als Schienen oder Führungen ausgebildet sein. Anschließend wird ein mechatronisches Funktionselement an den Ankern befestigt und damit ein Prothesenschaft zu einem mechatronischen Gesamtsystem komplettiert. Sofern eine distale Endkappe an dem Grundkörper angeordnet oder ausgebildet ist, ist in einer Weiterbildung die Befestigung des mechatronischen Funktionselementes an der Endkappe vorgesehen, insbesondere um eine Verlagerung in Proximalrichtung zu verhindern. Das Funktionselement ist als Modul ausgebildet und überdeckt oder füllt einen Bereich zwischen den einander gegenüberliegenden Schaftwandrändern aus.

Der Grundkörper kann mittels eines additiven Fertigungsverfahrens, beispielsweise nach Erfassen der Stumpfkontur und einer Anpassung der notwendigen Daten an die Belastungen und den Einsatzzweck erfolgen. Alternativ kann der Grundkörper im Rahmen eines Spritzgussverfahrens, aus einem thermoplastischen Material auf eine andere Art und Weise geformt oder über ein Laminierverfahren hergestellt werden. Grundsätzlich besteht auch die Möglichkeit eines direkten Anformens auf einem Modell, beispielsweise einem Abguss, oder dem Gliedmaßenstumpf direkt zu erzeugen. Auch hier können die Schaftwandränder oder die Schaftwandrandbereiche bei der Erzeugung des Grundkörpers ausgebildet oder nachträglich herausgearbeitet werden.

Die Ausgestaltung eines Grundkörpers beispielsweise um ein Gipsmodell herum, ist im Rahmen eines Laminierverfahrens oder eines Aufbringens eines thermoplastischen Materials erleichtert, wenn der Grundkörper mit einem geschlossenen Querschnitt ausgebildet wird. Das Gipsmodell oder das anderweitige Modell wird dabei im Umfang vollständig von dem Schaftwand material umgeben. Die Schaftwandränder werden dann durch Heraustrennen eines Segmentes des Grundkörpers ausgebildet.

Die Anker und der Grundkörper sind in einer Weiterbildung formschlüssig, kraftschlüssig und/oder stoffschlüssig miteinander verbunden, beispielsweise eingegossen, angeschraubt, angeschweißt, angenietet, eingeklemmt, eingehakt, eingeklebt, einlaminiert, anlaminiert, angenäht, eingeschoben und/oder aufgeschoben und gegebenenfalls nachträglich stoffschlüssig verbunden, beispielsweise durch Verschweißen, Verkleben oder ähnliches.

**In** einer Weiterbildung wird das mechatronische Funktionselement formschlüssig und/oder kraftschlüssig mit den Ankern verriegelt, um so eine reversible Befestigung und ein reversibles Lösen des jeweiligen Funktionselementes zu ermöglichen.

Durch die Ausgestaltung des mechatronischen Funktionselementes als ein vorgefertigtes Modul mit allen notwendigen Baugruppen und einer zentralen Recheneinheit kann eine optimale Abstimmung aller Komponenten, wie Energieversorgung, Sensorik, Aktuatorik und Steuerung oder Regelung erreicht werden. Mit dem Prothesenschaftsystem ist es möglich, eine aktive, situationsbedingte Volumeneinstellung eines individuellen Prothesenschaftes mit einer standardisierten Funktionsgruppe zu erzeugen. Darüber hinaus existieren standardisierte Schnittstellen zur Vernetzung mit mehreren mechatronischen Modulen. Beispielsweise können mehrere mechatronische Funktionselemente an einem Grundkörper zwischen zwei Schaftwandrändern eingesetzt werden, um entweder den Verstellweg zu vergrößern oder unterschiedliche Verstellungen zu ermöglichen. Ebenso kann eine Kommunikation zwischen unterschiedlichen Prothesenschaftsystemen oder zu einer Auswerteeinrichtung oder einem Orthopädietechniker aufgebaut werden, um Nutzungsdaten zu übermitteln und eine Auswertung zu ermöglichen.

Das Prothesenschaftsystem sieht eine Kombination aus einem vorgefertigten mechatronischen Funktionselement und gegebenenfalls individuellen, an die jeweilige Gliedmaße und den jeweiligen Patienten angepassten Komponenten eines Prothesenschaftes vor. Es ist möglich aufgrund der vordefinierten äußeren Abmessungen des mechatronischen Funktionselementes eine präzise Fertigung des Prothesenschaftes zu gewährleisten und dabei gleichzeitig eine individuelle Anpassbarkeit zu erreichen. Dadurch ist es nicht mehr notwendig, einen starren, umfänglich geschlossenen, individuellen Schaft zu erzeugen und Volumenschwankungen aufgrund einer verringerten Benutzungsdauer oder einer Veränderung der körperlichen Beschaffenheit des Prothesennutzers durch eine Anpassung eines Linersystems zu kompensieren. Die Fertigung des Prothesenschaftes kann erleichtert werden, da die Fertigungsgenauigkeit nicht mehr so hoch sein muss. Alle kritischen mechatronischen Schnittstellen können vorab industriell gefertigt werden, wodurch sich eine maximale Zuverlässigkeit während der Benutzung bei einer minimalen Baugröße ergeben.

Nachfolgend werden Ausführungsbeispiele und Aspekte der Erfindung anhand der beigefügten Figuren näher erläutert. Es zeigen:
- Figur 1 -: eine Einzelansicht eines mechatronischen Funktionselementes;
- Figur 2 -: eine Einzelansicht eines Grundkörpers;
- Figur 3 -: ein Prothesenschaftsystem mit Grundkörper und eingesetztem Funktionselement;
- Figur 4 -: ein Funktionselement mit verlagerten Ankern;
- Figur 5 -: eine Variante der Figur 3 in einem geöffneten Zustand;
- Figur 6 -: ein Grundkörper mit vergrößertem Durchmesser;
- Figur 7 -: das Funktionselement mit zusätzlichen Komponenten;
- Figur 8 -: das Prothesensystem und dessen Montage;
- Figur 9 -: eine Variante mit einem Funktionselement als Pelotte; sowie
- Figur 9a -: zwei Schnittdarstellungen entlang A-A der Figur 9.

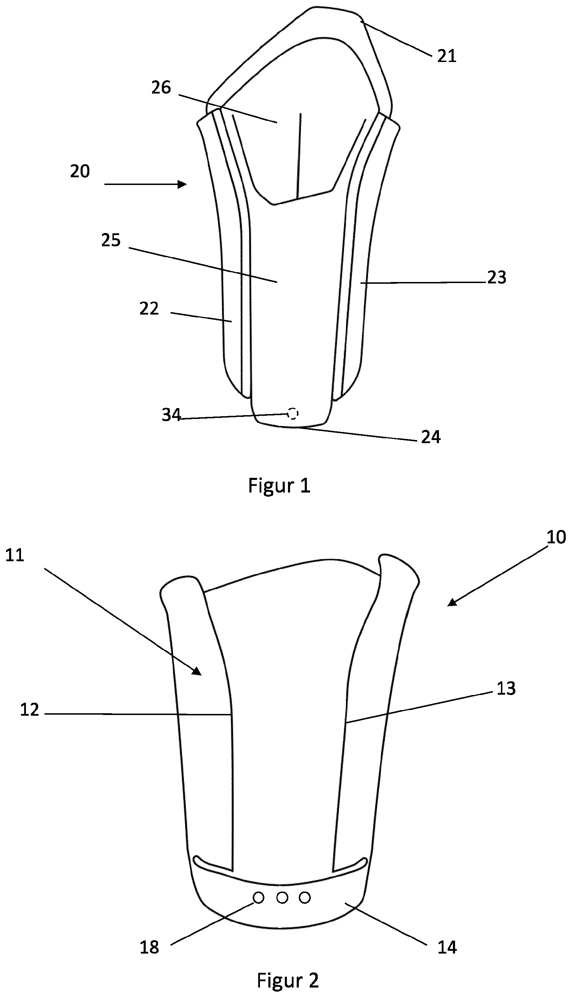

**In** der Figur 1 ist in einer Einzeldarstellung in der Frontalansicht ein Funktionselement 20 für ein Prothesenschaftsystem gezeigt, das einen Basiskörper 25 aufweist, der ein proximales Ende 21 und einen distalen Endbereich 24 aufweist. Innerhalb des Basiskörpers 25 sind mechanische und elektrische sowie elektronische Komponenten angeordnet, beispielsweise ein oder mehrere Energiespeicher, ein oder mehrere Sensoren, eine Steuerungseinheit, zumindest eine Kommunikationsschnittstelle und/oder zumindest ein Aktuator. Die Funktionsweise und die Komponenten werden weiter unten näher erläutert. Das mechatronische Funktionselement 20 weist in dem dargestellten Ausführungsbeispiel zwei Anker 22, 23 auf, die beweglich und verlagerbar an dem Basiskörper 25 befestigt sind. Die Anker 23 werden durch einen nicht dargestellten Aktuator relativ zueinander und relativ zu dem Basiskörper 25 verstellt und ermöglichen es, entweder aufgrund von Sensordaten oder aufgrund einer Betätigung von Tastern oder Schaltern in einem Bedienfeld 26, das im proximalen, frontalen Bereich des Basiskörpers 25 angeordnet oder ausgebildet ist, verstellt zu werden. Die Verstellung erfolgt entweder einzeln oder kombiniert. Der jeweilige Anker 22, 23 kann somit separat oder bevorzugt in entgegengesetzte Richtung zum gegenüberliegenden Anker verlagert werden. Neben einer geradlinigen Verlagerung in entgegengesetzte Richtungen können die Anker 22, 23 auch an ihren distalen und proximalen Endbereichen unterschiedliche Verlagerungswege ausführen, sodass im proximalen Bereich beispielsweise eine größere Verlagerung von dem Basiskörper 25 weg nach außen erfolgt als im distalen Bereich. Im distalen Endbereich 24 können Befestigungseinrichtungen 34 in Gestalt von Formschlusselementen oder Kraftschlusselementen, beispielsweise Vorsprüngen, Hintersprüngen, Klipse, Stege, Schnappeinrichtungen oder Klemmelemente angeordnet oder ausgebildet sein, um den Basiskörper an einem weiteren Element des Prothesenschaftsystems festzulegen. Das mechatronische Funktionselement 20 mit den Ankern 22, 23 kann industriell vorgefertigt werden und weist sämtliche sensorischen, elektrischen, elektronischen und mechanischen Komponenten auf, die für die vorgesehene Funktion notwendig sind. Dadurch ist es möglich, das mechatronische Funktionselement 20 als ein vorgefertigtes Modul bereitzustellen, das an einen jeweiligen Patienten oder einen individuellen Prothesenschaft angeordnet und daran befestigt werden kann, um über das mechatronische Funktionselement eine Anpassung und Individualisierung sowie Funktionserweiterung eines Prothesenschaftes zu ermöglichen.

Ein Grundkörper 10 eines Prothesenschaftes mit einer Schaftwand 11 ist in der Figur 2 dargestellt. Der Grundkörper 10 weist eine proximale Einstiegsöffnung und einen im Wesentlichen geschlossenen distalen Endbereich auf, der im dargestellten Ausführungsbeispiel als eine Endkappe 14 ausgebildet ist. An der Endkappe 14 können Befestigungseinrichtungen für weitere Prothesenkomponenten angeordnet sein, beispielsweise Aufnahmeadapter für ein Prothesengelenk oder für andere prothetische oder orthopädietechnische Komponenten wie Schienen, Antriebe, Prothesenhände, Prothesenfüße oder dergleichen. Innerhalb der durchgängig ausgebildeten Schaftwand 11 ist ein Ausschnitt ausgebildet, sodass zwei Schaftwandränder 12, 13 entstehen, die einander gegenüberliegen. Der Ausschnitt kann bei der Herstellung des Grundkörpers 10 erzeugt werden oder nach einer Herstellung eines zunächst im Umfang geschlossenen Grundkörpers 10 herausgetrennt werden. Das Heraustrennen kann durch Heraussägen, Schleifen, Bohren, Wasserstrahlschneiden oder andere Trennverfahren erfolgen. Der Ausschnitt erstreckt sich von dem proximalen Rand des Grundkörpers 10 in distale Richtung bis zu dem oberen Rand der distalen Endkappe 14 und erweitert sich dann umfänglich zu jeweils nach rechts und links abgehenden Schlitzen. Über die Schlitze wird eine Verlagerbarkeit der Schaftränder 12, 13 zueinander und voneinander weg ermöglicht. Innerhalb des Ausschnittes wird das in Figur 1 gezeigte Funktionselement 20 eingesetzt. Dazu ist der Ausschnitt entsprechend ausgebildet, das heißt, dass die Schaftwandränder 12, 13 so zueinander verlaufen und zueinander beabstandet sind, dass die Anker 22, 23 daran oder darin festgelegt werden können.

Im proximalen Bereich der Endkappe 14 sind Befestigungseinrichtungen 18 für das mechatronische Funktionselement 20 angeordnet oder ausgebildet. Die Befestigungseinrichtungen 18 sind beispielsweise Formschlusselemente oder Magnete, die korrespondierend zu den Befestigungseinrichtungen 34 des mechatronischen Funktionselementes 20 ausgebildet und angeordnet sind. Wenn das Funktionselement 20 in den Ausschnitt des Grundkörpers 10 eingeführt wird, können die Befestigungseinrichtungen 18, 34 miteinander in Eingriff treten und eine Verriegelung des Funktionselementes 20 an der Endkappe 14 ermöglichen.

Die Anker 22, 23 können rein mechanisch an dem Grundkörper 10 festgelegt werden, beispielsweise festgeklemmt, vernietet, verschraubt oder in Klemmschienen, die an den Ankern 22, 23 und/oder an den Schaftwandrändern 12, 13 angeordnet sind, befestigt werden. Alternativ oder ergänzend können die Anker 22, 23 an den Schaftwandrändern 12, 13 oder in den umfänglich daran angrenzenden Schaftwandbereichen stoffschlüssig festgelegt werden, beispielsweise durch Verkleben, Verschweißen oder andere stoffschlüssige Verbindungsverfahren.

Der Grundkörper 10 kann beispielsweise aus Faserverbundwerkstoffen auf einem abgeformten Modell eines Gliedmaßenstumpfes angeformt, mit Aufnahmeeinrichtungen für weitere prothetische Komponenten versehen und anschließend hinsichtlich des Ausschnittes so aufbereitet werden, dass die Schaftwandränder 12, 13 geeignet sind, mit den Ankern 22, 23 verbunden zu werden. Aufgrund der relativ zu dem Basiskörper 25 beweglichen Ausgestaltung der Anker 22, 23 kann eine nachträgliche Justierung leicht erfolgen, sodass eine geringere Fertigungsgenauigkeit ausreicht, um einen passgenauen Prothesenschaft herzustellen. Dazu ist die Innenseite des mechatronischen Funktionselementes 20, also diejenige Seite, die dem Patienten zugewandt ist, der grundsätzlichen Kontur der aufzunehmenden Gliedmaße entsprechend ausgebildet, insbesondere so gewölbt, dass eine möglichst vollständige Anlage an einer Gliedmaße oder einem Gliedmaßenstumpf erreicht werden kann. Der Grundkörper 10 kann auch in einem additiven Fertigungsverfahren auf Grundlage von Formdaten des Gliedmaßenstumpfes oder der Gliedmaße erstellt werden. Die Formdaten können insbesondere berührungslos durch Scannen erhalten und in ein entsprechendes 3D-Druckprogramm umgewandelt werden. Auf Basis dieser Daten wird dann der Grundkörper 10, vorteilhafterweise gleichzeitig mit dem Ausschnitt und gegebenenfalls mit entsprechenden Schaftwandrändern 12, 13 gefertigt. An den Schaftwandrändern 12, 13 können beispielsweise Verdickungen oder Leisten ausgebildet werden, die eine formschlüssige Verriegelung mit korrespondierenden Schienenelementen an den Ankern 22, 23 erleichtern oder ermöglichen.

Eine alternative Art und Weise der Herstellung des Grundkörpers 10 besteht darin, diesen im Rahmen eines Spritzgießverfahrens herzustellen, also einen vorkonfektionierten, standardisierten Prothesenschaftgrundkörper herzustellen, beispielsweise in verschiedenen Größenabstufungen, um dann den Ausschnitt einzuarbeiten oder bereits mit vorhandenen Ausschnitten einen solchen Grundkörper 10 spritzugießen. Ebenfalls ist es möglich, den Grundkörper 10 aus mehreren vorgefertigten Modulen zu fügen, sodass die Schaftwand 11 aus mehreren Komponenten besteht.

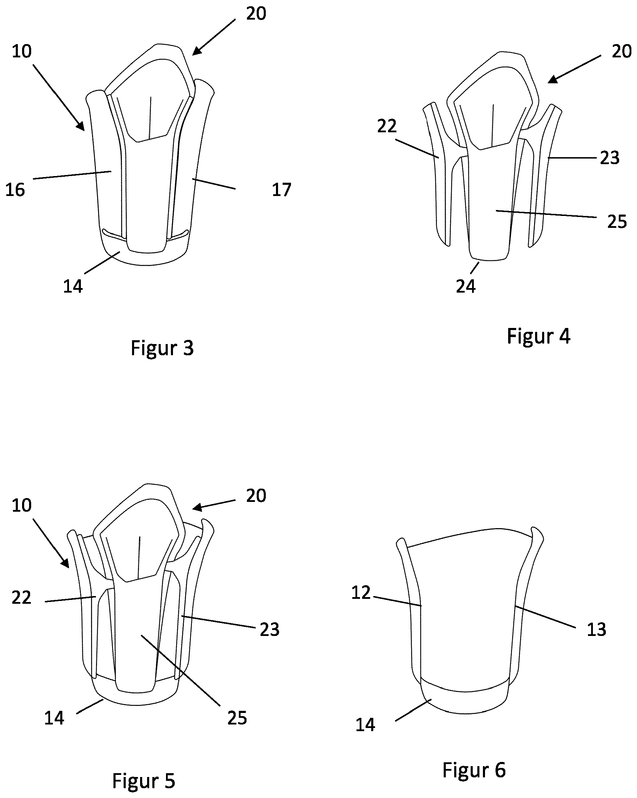

In der Figur 3 ist ein vollständig gefügtes Prothesenschaftsystem mit Grundkörper 10 und dem mechatronischen Funktionselement 20 gezeigt, bei dem die Anker 22, 23 an den Schaftwandrandbereichen 16, 17 beiderseits des Grundkörpers befestigt sind. Die Befestigung kann, wie oben ausgeführt, formschlüssig oder stoffschlüssig erfolgen, beispielsweise durch Einlaminieren, Eingießen, Einspritzen oder ähnliches. Das distale Endstück des Basiskörpers 25 ragt über den proximalen Rand der Endkappe 14 in Distalrichtung hinaus. Die seitlichen Schlitze dienen zur Erleichterung einer elastischen Verformung der Schaftwand 11 und ermöglichen ein Aufweiten und eine Volumenverringerung des Prothesenschaftes zur Anpassung an den jeweiligen Patienten.

Figur 4 zeigt das mechatronische Funktionselement mit maximal verstellten Ankern 22, 23, die von dem Basiskörper 25 maximal nach außen verlagert sind.

In der Figur 5 ist eine maximal aufgeweitete Stellung der Anker 22, 23 in dem montierten Zustand gezeigt. Der Basiskörper 25 ist mit der Endkappe 14 weiterhin verbunden, die einlaminierten, eingeschobenen, aufgeklebten, ein- oder angeschweißten oder auf andere Art und Weise an der Schaftwand 11 festgelegten Anker 22, 23 sind maximal auseinandergefahren, sodass sich die Schaftwandbereiche 16, 17 nach außen aufweiten, was durch die umlaufenden Schlitze proximal zu der Endkappe 14 erleichtert wird. In einem solchen Zustand befindet sich der Prothesenschaft, wenn beispielsweise der Nutzer des Prothesenschaftsystemes in den Prothesenschaft einsteigen oder aussteigen will. Auch zur Entlastung und zur Komforterhöhung kann eine solche geöffnete Stellung eingenommen werden.

In der Figur 6 ist der Grundkörper 10 mit dem deutlich vergrößerten Innendurchmesser und Innenvolumen mit den maximal auseinanderbewegten Schaftwandrändern 12, 13 dargestellt.

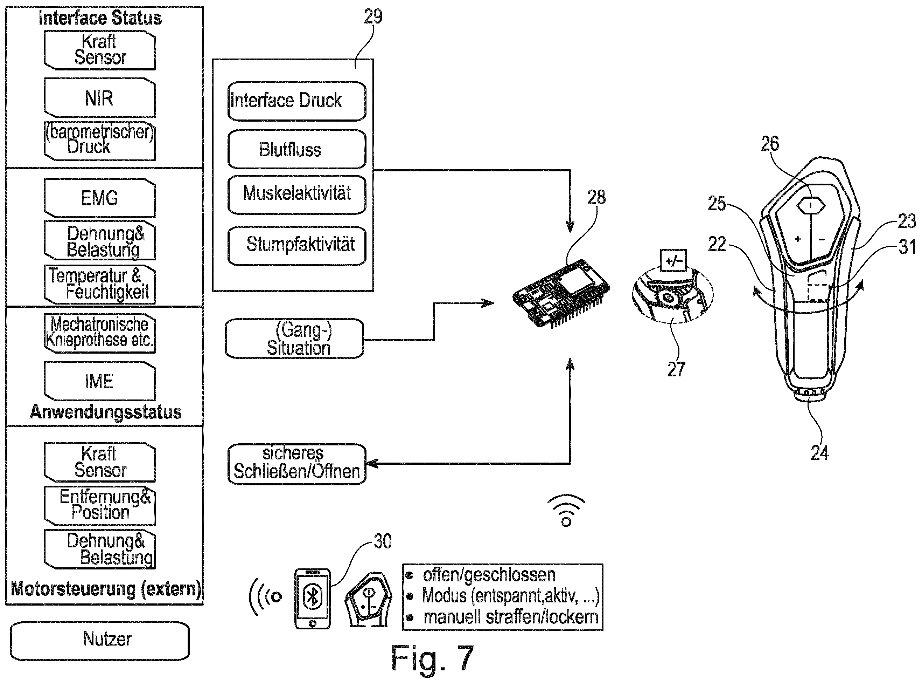

In der Figur 7 ist das mechatronische Funktionselement 20 und dessen Komponenten sowie die elektronische Anbindung näher dargestellt. Der Basiskörper 25 dient neben einer strukturellen Festigkeit für den übrigen Prothesenschaft auch als Gehäuse zur Aufnahme weiterer Komponenten, insbesondere eines Aktuators 27, der in Gestalt eines elektrischen Motors mit gegebenenfalls Getriebe und einem Zahnrad ausgebildet ist, in das auf gegenüberliegenden Seiten Zahnstangen eingreifen, die mit den Ankern 22, 23 verbunden sind. Wird der Aktuator 27 im Uhrzeigersinn betrieben, verfahren die Anker 22, 23 in einander entgegengesetzte Richtungen auseinander. Wird der Aktuator 27 in entgegengesetzte Richtung, also entgegen dem Uhrzeigersinn betrieben, fahren die beiden Anker 22, 23 wieder aufeinander zu in Richtung auf den Basiskörper 25. Diese Bewegung kann über das Bedienfeld 26 mit den Tasten ausgeführt werden. Ebenfalls ist es möglich, dass eine solche Aktuierung der Anker 22, 23 und damit eine Volumenveränderung auf der Grundlage von Sensordaten oder extern übermittelten Daten geschieht. Dazu ist in dem mechatronischen Funktionselement 20 eine Steuerungseinrichtung 28 angeordnet, die mit Sensoren 29 gekoppelt ist. Die Sensoren 29 können beispielsweise den Druck des Prothesenschaftes, einem Blutfluss, Muskelaktivitäten, Stumpfaktivitäten, eine Gangsituation oder auch eine Notfallsituation detektieren. Die Sensordaten werden der Steuerungseinrichtung 28 übermittelt. Dort ist ein entsprechendes Software-Programm hinterlegt, das in der CPU der Steuerungseinrichtung 28 verarbeitet wird und zur Aktivierung oder Deaktivierung des Aktuators 27 führt. Darüber hinaus ist eine Kommunikationsschnittstelle 30 zu der Steuerungseinrichtung 28 vorgesehen, über die einerseits Daten an beispielsweise eine mobile Datenverarbeitungseinrichtung, wie Mobiltelefon, Tablet, Computer oder auch über das Internet an eine externe Datenverarbeitungseinrichtung geleitet werden kann. Der Nutzer oder ein Orthopädietechniker kann dann den jeweiligen Status erkennen und Daten über eine drahtlose Verbindung empfangen oder wieder zurücksenden, beispielsweise um den Prothesenschaft zu öffnen oder zu schließen, eine Massagefunktion auszuüben, eine belastungsangepasste Verringerung oder Vergrößerung des Prothesenschaftes zu bewirken oder dergleichen. Die Kommunikationsschnittstelle 30 ermöglicht eine Vernetzung des Prothesenschaftsystems mit externen Einrichtungen, weiteren Prothesenkomponenten, medizinischen Auswertstellen, Datenbanken und/oder Herstellern, um notwendige Anpassungen vorzunehmen oder Daten zur Bewegungsanalyse zu gewinnen. Der Orthopädietechniker kann beispielsweise beim Benutzen einer Prothese anhand der Drucksensoren erkennen, ob und wo welche Belastung auf den Gliedmaßenstumpf einwirken, ob eine ausreichende Durchblutung stattfindet, wie hoch die Temperatur ist und ob über ein Muskelaktivität das Volumen verändert werden soll oder nicht.

Eine automatische Adaption des Prothesenschaftes kann beispielsweise beim Einsteigen in den Prothesenschaft, beim Ausziehen des Prothesenschaftes, beim Hinsetzen, Autofahren, während der Standphase oder der Schwungphase beim Gehen, zur Rotationsstabilisierung beim Fersenauftritt oder zum Ausgleich von Volumenzunahmen oder -abnahmen erfolgen.

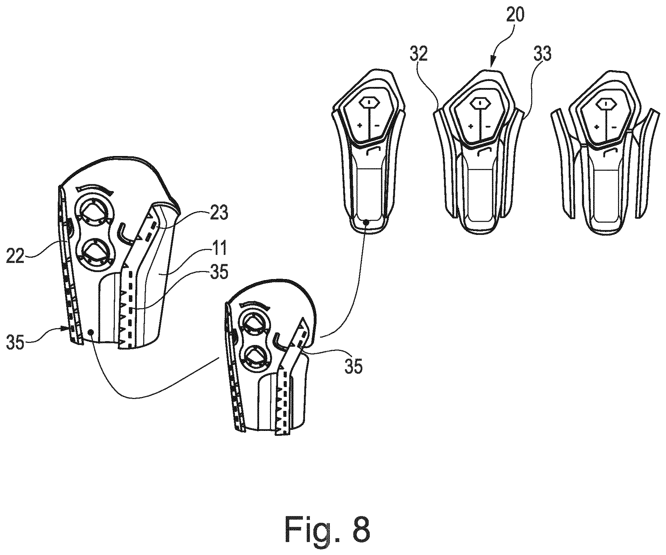

In der Figur 8 ist eine Variante des Prothesenschaftsystems gezeigt. In der oberen rechten Darstellung ist das mechatronische Funktionselement 20 gezeigt, bei dem jedoch keine Anker an dem Basiskörper 25 verlagerbar angeordnet sind, sondern Befestigungseinrichtungen 32, 33, die eine reversible, mechanische Verriegelung mit den Ankern 22, 23 ermöglichen. Die Befestigungseinrichtungen 32, 33 weisen insbesondere Formschlusselemente und/oder Kraftschlusselemente auf, mit denen es möglich ist, eine mechanische, wiederholt lösbare und verriegelbare Befestigung des Funktionselementes 20 an dem Grundkörper 10 zu ermöglichen. Der Grundkörper 10 ist in der linken Darstellung gezeigt, an dem bereits an den Schaftwandrändern die Anker 22, 23 mit daran ausgeformten Formschlusselementen 35 dargestellt sind. Die Schienen 22, 23 sind korrespondierend zu den Befestigungseinrichtungen 32, 33 geformt und erlauben eine mechanische Verriegelung der Schienen 22, 23 mit den Befestigungseinrichtungen 32, 33. Dazu werden die Befestigungseinrichtungen 32, 33 auf die Schienen 22, 23 aufgeschoben, aufgeklipst und damit formschlüssig und/oder kraftschlüssig verriegelt, gegebenenfalls noch gesichert über Schrauben, Befestigungseinrichtungen, Verriegelungselemente oder dergleichen. Die Schienen 22, 23 verlaufen ebenfalls in Proximal-Distal-Richtung und ermöglichen ein Einschieben des Funktionselementes 20 von oben und eine mechanische Verriegelung, sodass über den gesamten Umfang ein innerhalb des Prothesenschaftes aufgenommener Gliedmaßenstumpf sicher gehalten wird. Das mechatronische Funktionselement 20 ist insbesondere lateral oder frontal an dem Grundkörper 10 angeordnet, sodass eine leichte Zugänglichkeit gegeben ist. Dies ist insbesondere bei Prothesenschäften für Prothesenkniegelenke zur Aufnahme von Oberschenkelstümpfen vorteilhaft. Auch bei Unterschenkelstümpfen kann eine solche Anordnung sinnvoll sein, da an diesen Stellen das etwas vergrößerte Volumen des Prothesenschaftes aufgrund der integrierten mechanischen, elektronischen und elektrischen Komponenten bei der Benutzung nicht stört.

Mit dem Prothesenschaftsystem wird über eine standardisierte oder individuelle Schaftbasis in Gestalt des Grundkörpers 10 in Verbindung mit verstellbaren Ankern 22, 23 ein auto-adaptives Prothesenschaftsystem mit einer Funktionserweiterung im Vergleich zu herkömmlichen Prothesenschaftsystemen erreicht. Es können auch mehrere Funktionselemente 20 an einem Grundkörper 10 angeordnet sein, beispielsweise einander gegenüberliegend, um eine größere Variation an Volumenverstellungen zu ermöglichen. Darüber hinaus ist es möglich, gezielte Manipulationen spezifischer Anlageflächen zu erreichen. Die Sensoren 29 sind vorteilhafterweise innerhalb des Funktionselementes 20 angeordnet, die Steuerungseinrichtung 28 kann auch mit weiteren Sensoren oder externen Datenquellen gekoppelt werden, um den Aktuator 27 zu bewegen. Mehrere Aktuatoren 27 können vorhanden sein, um eine individuelle Verstellung entweder der Anker 22, 23 zueinander oder eine Verlagerung in mehrere Richtungen zu erreichen. Die Anker 22, 23 können verdreht werden, ebenso kann neben einer seitlichen Bewegung eine Bewegung in Proximal-Distal-Richtung und/oder nach außen oder innen, jeweils gesehen vom Stumpf erfolgen.

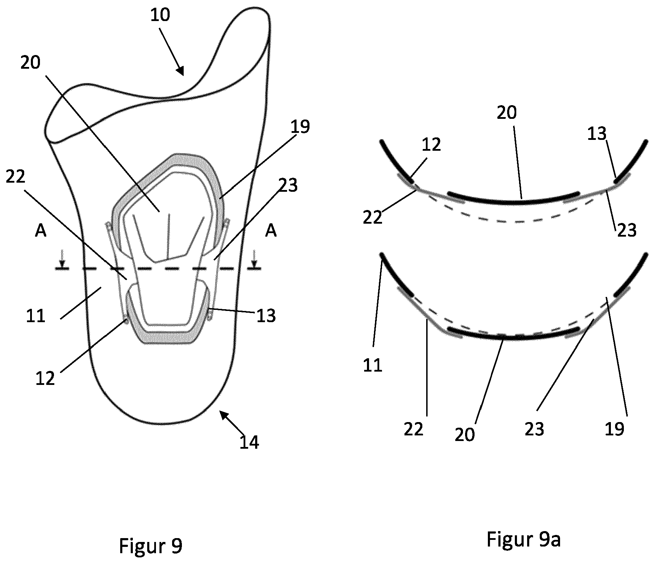

In der Figur 9 ist in einer perspektivischen, schematischen Darstellung ein Prothesenschaft mit einem Grundkörper 10 und einer Schaftwand 11 dargestellt. Der Grundkörper 10 weist eine konische Grundform auf, die sich von einer distalen Endkappe 14 in proximaler Richtung aufweitet. Der Grundkörper 10 weist einen im Wesentlichen geschlossenen Querschnitt auf und umgibt im montierten, angezogenen Zustand den nicht dargestellten Gliedmaßenstumpf vollständig. An der distalen Endkappe 14 sind nicht dargestellte Befestigungseinrichtungen für weitere Prothesenkomponenten vorhanden, beispielsweise Gelenke oder Funktionselemente wie Prothesenhände, Prothesenfüße oder dergleichen. Innerhalb der Schaftwand 11 ist ein Ausschnitt 19 ausgebildet, der entweder bei dem Urformen des Grundkörpers 10 ausgebildet wurde oder nachträglich in einen ursprünglich den Stumpf vollständig umgebenen Grundkörper 10 eingearbeitet wurde. Der Ausschnitt 19 kann beispielsweise herausgeschnitten, herausgesägt oder durch andere Trennverfahren erzeugt werden. An dem Ausschnitt 19 sind Schaftränder 12, 13 ausgebildet, die einander gegenüberliegen. Der Ausschnitt 19 ist asymmetrisch ausgebildet und weist eine größere Erstreckung in proximal-distal Richtung als in Umfangsrichtung auf. Die beiden im Wesentlichen in proximal-distal-Richtung verlaufenden Schaftwandränder 12, 13 liegen einander gegenüber, auch wenn sie nicht parallel zueinander orientiert verlaufen. Die beiden Schaftwandränder 12, 13 können eingefasst sein, ebenso können die übrigen Ränder des Ausschnittes 19 bearbeitet oder eingefasst sein, beispielsweise können an oder in der Schaftwand 11 Befestigungseinrichtungen oder Formschlusselemente zum Festlegen der Anker 22, 23 angeordnet oder ausgebildet sein. Der Ausschnitt 19 kann durch eine Folie, ein Polster, ein Textil oder eine andere, flexible gegebenenfalls elastische Abdeckung teilweise oder vollständig verschlossen sein. Die Orientierung der Schaftwandränder 12, 13 ist frei wählbar, aufgrund der größeren Variabilität in Umfangsrichtung erscheint eine Orientierung in proximal-distal-Richtung als die wahrscheinlich häufigste Wahl

Außen an der Schaftwand 11 sind an den Schaftwandrändern 12, 13 bzw. Schaftrandwandbereichen zwei Anker 22, 23 festgelegt, die formschlüssig und reversibel an dem Grundkörper 10 festlegbar sind. Die Anker 22, 23 sind beweglich in dem mechatronischen Funktionselement 20 gelagert, das in dem dargestellten Ausführungsbeispiel als eine sogenannte Pelotte ausgebildet ist. Innerhalb des mechatronischen Funktionselementes 20 sind die oben beschriebenen, nicht näher dargestellten Komponenten wie Energiespeicher, Sensoren, Sender, Empfänger, Energie- und Datenschnittstellen, Steuerungseinrichtungen, Prozessoren, Datenspeicher, Aktuatoren wie Motoren oder dergleichen integriert.

In der Figur 9a sind zwei Zustände mit unterschiedlich positionierten Ankern 22, 23 relativ zu dem Zentralkörper des mechatronischen Funktionselementes 20 dargestellt. In der oberen Figur sind die Anker 22, 23 aufeinander zu bewegt, sodass eine Verkürzung der effektiven Länge zwischen den Schafträndern oder Rändern 12, 13 des Ausschnittes 19 bewirkt wird. Die Schaftränder 12, 13 sind in dem dargestellten Ausführungsbeispiel nicht oder nur unwesentlich aufeinander zu verlagert, sodass der Zentralkörper des mechatronischen Funktionselementes 20 in das Innere des Grundkörpers 10 verlagert wird. Die Krümmungslinie eines ununterbrochenen Grundkörpers 10 ist durch die gestrichelte Linie dargestellt. In einem solchen Zustand wird ein erhöhter Druck durch den Zentralkörper auf das Innere des Grundkörpers 10 ausgeübt, also auf den darin aufgenommenen Stumpf.

Werden die beiden Anker 22, 23 in entgegengesetzte Richtung bewegt, also voneinander weg verlagert, vergrößert sich die effektive Länge des mechatronischen Funktionselementes 20 zwischen den beiden Rändern 12, 13 in dem Bereich des Ausschnittes 19, sodass der Zentralkörper nach außen bewegt wird. Dies ist in der unteren Darstellung der Figur 9a gezeigt. Der innere Umfang im Bereich des Ausschnittes 19, der durch das mechatronische Funktionselement 20 zumindest teilweise abgedeckt wird, entspricht dabei im Wesentlichen der fortgesetzten Umfangslinie der Schaftwand 11 des Grundkörpers 10. Sollte der Stumpf eine weitere Entlastung benötigen, kann die Verfahrbewegung der Anker 22, 23 nach außen relativ zu dem Zentralkörper des mechatronischen Funktionselementes 20 fortgesetzt werden. Werden die Antriebe oder Aktuatoren für die Verlagerung des Zentralkörpers relativ zu den Ankern 22 gleichsinnig betrieben, kann der Zentralkörper zwischen den Ankern 22, 23 bzw. zwischen den Rändern 12, 13 des Ausschnittes 19 verschoben werden. Gleiches gilt selbstverständlich auch, wenn durch den Ausschnitt oder durch die Schaftränder 12, 13 ein Ausschnitt ausgebildet wird, der sich bis zum proximalen Rand des Grundkörpers 10 erstreckt.

## Patentansprüche

1. Prothesenschaftsystem mit einem Grundkörper (10) mit einer Schaftwand (11), die im angelegten Zustand eine Gliedmaße oder einen Gliedmaßenstumpf zumindest teilweise umgibt und zumindest zwei sich einander gegenüberliegende Schaftwandränder (12, 13) oder Schaftwandbereiche (16, 17) aufweist, mit zwei Ankern (22, 23), wobei je ein Anker (22, 23) in einem Schaftwandrand (12, 13) oder an einem Schaftwandbereich (16, 17) angeordnet ist, sowie durch ein mechatronisches Funktionselement (20), das an den Ankern (22, 23) befestigt ist, **dadurch gekennzeichnet, dass** das Funktionselement (20) als Modul ausgebildet ist, das einen Bereich zwischen den einander gegenüberliegenden Schaftwandrändern (12, 13) überdeckt oder diesen ausfüllt.

2. Prothesenschaftsystem nach Anspruch 1, **dadurch gekennzeichnet dass** das mechatronische Funktionselement (20) zumindest einen Energiespeicher (31), einen Sensor (29), eine Steuerungseinheit (28), eine Kommunikationsschnittstelle (30) und/oder einen Aktuator (27) aufweist.

3. Prothesenschaftsystem nach Anspruch 1 oder 2, **dadurch gekennzeichnet, dass** das mechatronische Funktionselement (20) reversibel lösbar an den Ankern (22, 23) festlegbar ausgebildet ist.

4. Prothesenschaftsystem nach einem der voranstehenden Ansprüche, **dadurch gekennzeichnet, dass** das mechatronische Funktionselement (20) zum Bewegen der Anker (22, 23) zueinander oder zur Verlagerung relativ zu den Ankern (22, 23) oder der Schaftwand (11) ausgebildet ist.

5. Prothesenschaftsystem nach einem der voranstehenden Ansprüche, **dadurch gekennzeichnet, dass** die Anker (22, 23) als Formschlusselemente und/oder Kraftschlusselemente ausgebildet sind.

6. Prothesenschaftsystem nach einem der voranstehenden Ansprüche, **dadurch gekennzeichnet, dass** die Anker (22, 23) in oder an dem Grundkörper (10) formschlüssig, kraftschlüssig und/oder stoffschlüssig befestigt sind.

7. Prothesenschaftsystem nach einem der voranstehenden Ansprüche, **dadurch gekennzeichnet, dass** der Grundkörper (10) individuell geformt, aus vorgefertigten Modulen oder vorgeformt ausgebildet ist.

8. Prothesenschaftsystem nach einem der voranstehenden Ansprüche, **dadurch gekennzeichnet, dass** die Schaftwandränder (12, 13) durch ein Trennverfahren erzeugt sind.

9. Prothesenschaftsystem nach einem der voranstehenden Ansprüche, **dadurch gekennzeichnet, dass** die Schaftwandränder (12, 13) beabstandet zueinander und zumindest abschnittsweise entlang aneinander ausgerichtet oder sich konisch oder gekrümmt in Längserstreckung des Grundkörpers (10) aufweiten.

10. Prothesenschaftsystem nach einem der voranstehenden Ansprüche, **dadurch gekennzeichnet, dass** die Schaftwandränder (12, 13) in proximal-distal-Richtung oder schräg zu der proximal-distal-Richtung orientiert verlaufen.

11. Prothesenschaftsystem nach einem der voranstehenden Ansprüche, **dadurch gekennzeichnet, dass** der Grundkörper (10) spritzgegossen, aus thermoplastischem Material geformt, additiv gefertigt oder laminiert aufgebaut ist.

12. Prothesenschaftsystem nach einem der voranstehenden Ansprüche, **dadurch gekennzeichnet, dass** der Grundkörper (10) eine konische Grundform, eine konische Grundform mit einem offenen Querschnitt oder mit einem Ausschnitt (19) aufweist.

13. Prothesenschaftsystem nach einem der voranstehenden Ansprüche, **dadurch gekennzeichnet, dass** an dem Grundkörper (10) eine Endkappe (14) angeformt oder befestigt ist.

14. Prothesenschaftsystem nach Anspruch 13, **dadurch gekennzeichnet, dass** an der Endkappe (14) Befestigungseinrichtungen (18) für das mechatronische Funktionselement (20) angeordnet sind.

15. Prothesenschaftsystem nach einem der voranstehenden Ansprüche, **dadurch gekennzeichnet, dass** die Schaftwandränder (12, 13) elastisch zueinander verlagerbar und/oder die Schaftwand (11) reversibel verformbar ausgebildet sind.

16. Prothesenschaftsystem nach einem der voranstehenden Ansprüche, **dadurch gekennzeichnet, dass** an dem mechatronischen Funktionselement (20) Befestigungseinrichtungen (32, 33) für die Anker (22, 23) angeordnet oder ausgebildet sind.

17. Prothesenschaftsystem nach einem der voranstehenden Ansprüche, **dadurch gekennzeichnet, dass** das mechatronische Funktionselement (20) zwischen den Schaftwandrändern (12, 13), in einem Ausschnitt der Schaftwand (11) oder an der Außenseite der Schaftwand (11) angeordnet ist.

18. Verfahren zum Herstellen eines Prothesenschaftsystem nach einem der voranstehenden Ansprüche, das die Schritte aufweist:
- Erzeugen eines Grundkörpers (10) mit einer Schaftwand (11) und Schaftwandrändern (12, 13)r
- Befestigen oder Ausbilden von Ankern (22, 23) an den Schaftwandrändern (12, 13) oder Schaftwandrandbereichen (16, 17)
- Befestigen eines mechatronischen Funktionselementes (20) an den Ankern (22, 23)
- Überdecken oder Ausfüllen eines Bereiches zwischen den einander gegenüberliegenden Schaftwandrändern (12, 13) durch das mechatronische Funktionselement (20).

19. Verfahren nach Anspruch 18, **dadurch gekennzeichnet, dass** der Grundkörper (10) mittels eines additiven Fertigungsverfahrens, spritzgegossen, aus thermoplastischem Material geformt, laminiert oder eines direkten Anformens auf einem Modell oder einem Gliedmaßenstumpf erzeugt wird.

20. Verfahren nach Anspruch 18 oder 19, **dadurch gekennzeichnet, dass** zunächst ein Grundkörper (10) mit einem geschlossenen Querschnitt ausgebildet wird, aus dem unter Ausbildung der Schaftwandränder (12, 13) ein Segment herausgetrennt wird.

21. Verfahren nach einem der Ansprüche 18 bis 20, **dadurch gekennzeichnet, dass** die Anker (22, 23) und der Grundkörper (10) formschlüssig, kraftschlüssig und/oder stoffschlüssig miteinander verbunden werden.

22. Verfahren nach einem der Ansprüche 18 bis 21, **dadurch gekennzeichnet, dass** das mechatronische Funktionselement (20) formschlüssig und/oder kraftschlüssig mit den Ankern (22, 23) verriegelt wird.

## Claims

1. A prosthesis socket system having a main body (10) with a socket wall (11), which in the applied state surrounds a limb or a limb stump at least partially and has at least two opposite socket wall edges (12, 13) or socket wall regions (16, 17), with two anchors (22, 23), whereas each anchor (22, 23) being arranged in a socket wall edge (12, 13) or on a socket wall region (16, 17), and by a mechatronic functional element (20), which is fastened to the anchors (22, 23), **characterized in that** the functional element (20) is designed as a module that covers or fills an area between the opposite socket wall edges (12, 13).

2. The prosthesis socket system as claimed in claim 1, **characterized in that** the mechatronic functional element (20) has at least one energy storage device (31), a sensor (29), a control unit (28), a communication interface (30) and/or an actuator (27).

3. The prosthesis socket system as claimed in claim 1 or 2, **characterized in that** the mechatronic functional element (20) is designed to be able to be reversibly fastened releasably to the anchors (22, 23).

4. The prosthesis socket system as claimed in one of the preceding claims, **characterized in that** the mechatronic functional element (20) is designed for moving the anchors (22, 23) toward each other or for shifting relative to the anchors (22, 23) or the socket wall (11).

5. The prosthesis socket system as claimed in one of the preceding claims, **characterized in that** the anchors (22, 23) are designed as form-fit elements and/or force-fit elements.

6. The prosthesis socket system as claimed in one of the preceding claims, **characterized in that** the anchors (22, 23) are fastened in or on the main body (10) in a form-fitting, force-fitting and/or cohesively bonded manner.

7. The prosthesis socket system as claimed in one of the preceding claims, **characterized in that** the main body (10) is individually shaped from prefabricated modules or preformed.

8. The prosthesis socket system as claimed in one of the preceding claims, **characterized in that** the socket wall edges (12, 13) are produced by a separation method.

9. The prosthesis socket system as claimed in one of the preceding claims, **characterized in that** the socket wall edges (12, 13) are spaced apart from each other and at least partially aligned along each other or widen conically or curve in a longitudinal extent of the main body (10).

10. The prosthesis socket system as claimed in one of the preceding claims, **characterized in that** the socket wall edges (12, 13) are oriented in the proximal-distal direction or obliquely to the proximal-distal direction.

11. The prosthesis socket system as claimed in one of the preceding claims, **characterized in that** the main body (10) is injection-molded, formed of thermoplastic material, additively manufactured or laminated.

12. The prosthesis socket system as claimed in one of the preceding claims, **characterized in that** the main body (10) has a conical basic shape, a conical basic shape with an open cross section or with a cutout (19).

13. The prosthesis socket system as claimed in one of the preceding claims, **characterized in that** an end cap (14) is integrally formed or fastened on the main body (10).

14. The prosthesis socket system as claimed in claim 13, **characterized in that** fastening devices (18) for the mechatronic functional element (20) are arranged on the end cap (14).

15. The prosthesis socket system as claimed in one of the preceding claims, **characterized in that** the socket wall edges (12, 13) are designed to be elastically displaceable toward each other and/or the socket wall (11) is designed to be reversibly deformable.

16. The prosthesis socket system as claimed in one of the preceding claims, **characterized in that** fastening devices (32, 33) for the anchors (20, 23) are arranged or formed on the mechatronic functional element (22).

17. The prosthesis socket system as claimed in one of the preceding claims, **characterized in that** the mechatronic functional element (20) is arranged between the socket wall edges (12, 13), in a cutout of the socket wall (11) or on the outside of the socket wall (11).

18. A method for producing a prosthesis socket system as claimed in one of the preceding claims, which comprises the steps of:
- producing a main body (10) with a socket wall (11) and socket wall edges (12, 13),
- fastening or forming anchors (22, 23) on the socket wall edges (12, 13) or socket wall edge regions (16, 17),
- fastening a mechatronic functional element (20) to the anchors (22, 23)
- covering of filling of an area between the opposite socket wall edges (12, 13) by the mechatronic functional element (20).

19. The method as claimed in claim 18, **characterized in that** the main body (10) is produced by means of an additive manufacturing process, injection-molded, formed from thermoplastic material, laminated or directly formed on a model or a limb stump.

20. The method as claimed in claim 18 or 19, **characterized in that** a main body (10) with a closed cross section is initially formed, from which a segment is separated to form the socket wall edges (12, 13).

21. The method as claimed in one of claims 18 to 20, **characterized in that** the anchors (22, 23) and the main body (10) are connected to each other in a form-fitting, force-fitting and/or cohesively bonded manner.

22. The method as claimed in one of claims 18 to 21, **characterized in that** the mechatronic functional element (20) is locked onto the anchors (22, 23) in a form-fitting and/or force-fitting manner.

## Revendications

1. Système d'emboîture de prothèse comprenant un corps de base (10) muni d'une paroi d'emboîture (11) qui, à l'état appliqué, entoure au moins partiellement un membre ou un moignon de membre et qui présente au moins deux bordures de paroi d'emboîture (12, 13) ou zones de paroi d'emboîture (16, 17) opposées l'une à l'autre, et comprenant deux ancrages (22, 23), chaque ancrage (22, 23) étant disposé dans une bordure de paroi d'emboîture (12, 13) ou sur une zone de paroi d'emboîture (16, 17), ainsi qu'un élément fonctionnel mécatronique (20) qui est fixé aux ancrages (22, 23),
**caractérisé en ce que** l'élément fonctionnel (20) est réalisé comme un module qui recouvre ou remplit une zone située entre les bordures de paroi d'emboîture (12, 13) opposées l'une à l'autre.

2. Système d'emboîture de prothèse selon la revendication 1,
**caractérisé en ce que** l'élément fonctionnel mécatronique (20) comprend au moins un accumulateur d'énergie (31), un capteur (29), une unité de commande (28), une interface de communication (30) et/ou un actionneur (27).

3. Système d'emboîture de prothèse selon la revendication 1 ou 2,
**caractérisé en ce que** l'élément fonctionnel mécatronique (20) est réalisé pour être immobilisé de manière amovible et réversible aux ancrages (22, 23).

4. Système d'emboîture de prothèse selon l'une des revendications précédentes,
**caractérisé en ce que** l'élément fonctionnel mécatronique (20) est réalisé pour déplacer les ancrages (22, 23) l'un vers l'autre ou pour se déplacer par rapport aux ancrages (22, 23) ou à la paroi d'emboîture (11).

5. Système d'emboîture de prothèse selon l'une des revendications précédentes,
**caractérisé en ce que** les ancrages (22, 23) sont réalisés comme des éléments à liaison par complémentarité de forme et/ou des éléments à liaison par adhérence.

6. Système d'emboîture de prothèse selon l'une des revendications précédentes,
**caractérisé en ce que** les ancrages (22, 23) sont fixés dans ou sur le corps de base (10) par complémentarité de forme, par adhérence et/ou par liaison de matière.

7. Système d'emboîture de prothèse selon l'une des revendications précédentes,
**caractérisé en ce que** le corps de base (10) est formé individuellement, réalisé à partir de modules préfabriqués, ou préformé.

8. Système d'emboîture de prothèse selon l'une des revendications précédentes,
**caractérisé en ce que** les bordures de paroi d'emboîture (12, 13) sont créées par un procédé de séparation.

9. Système d'emboîture de prothèse selon l'une des revendications précédentes,
**caractérisé en ce que** les bordures de paroi d'emboîture (12, 13) sont espacées l'une de l'autre et orientées l'une le long de l'autre au moins localement ou s'évasent de manière conique ou courbée selon l'extension longitudinale du corps de base (10).

10. Système d'emboîture de prothèse selon l'une des revendications précédentes,
**caractérisé en ce que** les bordures de paroi d'emboîture (12, 13) s'étendent dans la direction proximale-distale ou en oblique par rapport à la direction proximale-distale.

11. Système d'emboîture de prothèse selon l'une des revendications précédentes,
**caractérisé en ce que** le corps de base (10) est moulé par injection, formé à partir d'un matériau thermoplastique, réalisé par fabrication additive ou avec une structure stratifiée.

12. Système d'emboîture de prothèse selon l'une des revendications précédentes,
**caractérisé en ce que** le corps de base (10) présente une forme de base conique, une forme de base conique avec une section transversale ouverte ou avec une découpe (19).

13. Système d'emboîture de prothèse selon l'une des revendications précédentes,
**caractérisé en ce qu'**un capuchon d'extrémité (14) est moulé ou fixé sur le corps de base (10).

14. Système d'emboîture de prothèse selon la revendication 13, **caractérisé en ce que** des dispositifs de fixation (18) pour l'élément fonctionnel mécatronique (20) sont disposés sur le capuchon d'extrémité (14).

15. Système d'emboîture de prothèse selon l'une des revendications précédentes,
**caractérisé en ce que** les bordures de paroi d'emboîture (12, 13) peuvent être déplacées élastiquement l'une par rapport à l'autre et/ou la paroi d'emboîture (11) est réalisée de façon à être déformable de manière réversible.

16. Système d'emboîture de prothèse selon l'une des revendications précédentes,
**caractérisé en ce que** des dispositifs de fixation (32, 33) pour les ancrages (22, 23) sont disposés ou réalisés sur l'élément fonctionnel mécatronique (20).

17. Système d'emboîture de prothèse selon l'une des revendications précédentes,
**caractérisé en ce que** l'élément fonctionnel mécatronique (20) est disposé entre les bordures de paroi d'emboîture (12, 13), dans une découpe de la paroi d'emboîture (11) ou sur la face extérieure de la paroi d'emboîture (11).

18. Procédé de fabrication d'un système d'emboîture de prothèse selon l'une des revendications précédentes, comprenant les étapes consistant à :
- créer un corps de base (10) muni d'une paroi d'emboîture (11) et de bordures de paroi d'emboîture (12, 13),
- fixer ou réaliser des ancrages (22, 23) sur les bordures de paroi d'emboîture (12, 13) ou les zones de bordure de paroi d'emboîture (16, 17),
- fixer un élément fonctionnel mécatronique (20) aux ancrages (22, 23),
- recouvrir ou remplir une zone située entre les bordures de paroi d'emboîture (12, 13) opposées l'une à l'autre par l'élément fonctionnel mécatronique (20).

19. Procédé selon la revendication 18,
**caractérisé en ce que** le corps de base (10) est fabriqué au moyen d'un procédé de fabrication additive, moulé par injection, formé à partir d'un matériau thermoplastique, stratifié ou créé par moulage direct sur un modèle ou un moignon de membre.

20. Procédé selon la revendication 18 ou 19,
**caractérisé en ce que** d'abord un corps de base (10) à section transversale fermée est réalisé, à partir duquel un segment est séparé en réalisant les bordures de paroi d'emboîture (12, 13).

21. Procédé selon l'une des revendications 18 à 20,
**caractérisé en ce que** les ancrages (22, 23) et le corps de base (10) sont reliés entre eux par complémentarité de forme, par adhérence et/ou par liaison de matière.

22. Procédé selon l'une des revendications 18 à 21,
**caractérisé en ce que** l'élément fonctionnel mécatronique (20) est verrouillé par complémentarité de forme et/ou par adhérence aux ancrages (22, 23).
